Europäisches Patentamt

**European Patent Office**

**Office européen des brevets**

(19)

(11) Publication number: **0 105 989**
A1

# EUROPEAN PATENT APPLICATION

(12)

(21) Application number: **82305473.9**

(51) Int. Cl.³: **A 61 M 5/14,** F 16 K 7/08

(22) Date of filing: **14.10.82**

(43) Date of publication of application: **25.04.84**
Bulletin 84/17

(84) Designated Contracting States: **AT BE CH DE FR IT LI
LU NL SE**

(71) Applicant: **BESPAK INDUSTRIES LIMITED, Bergen Way
North Lynn Industrial Estate, King's Lynn Norfolk
PE30 2JG (GB)**

(72) Inventor: **Barnes, Paul, 60 West Fields, Narborough
Norfolk (GB)**
Inventor: **Reed, Norman Leonard, 29 Ullswater Avenue
South Wootton, King's Lynn Norfolk (GB)**

(74) Representative: **Alexander, Thomas Bruce et al, Boult,
Wade & Tennant 27 Furnival Street, London EC4A 1PQ
(GB)**

(54) **Flow regulating apparatus.**

(57) A flow regulating apparatus is provided which is particularly useful in the field of medical administration of fluid to the body. The regulating apparatus comprises a housing (11) which has a cylindrical protrusion (20, 21) at either end so that the housing may be inserted into a tube of, for example, a medical infusion set. A piece of tubing (10) within the housing extends between the protrusions and one of the protrusions is mounted on a annular disc (12) so that it may be rotated relative to the other end of the housing. This rotation is controlled by a rachet mechanism (17) and rotation of the rachet causes the tubing to twist and thereby controls the rate of fluid flow through the tubing. The rate of flow may be controlled between a full bore rate and a zero flow rate when the tubing is twisted so that its bore is fully occluded.

ACTORUM AG

1.

## FLOW REGULATING APPARATUS

The present invention relates to apparatus for regulating the flow of fluent material. The invention is particularly useful in the field of medical administration of fluid to the body, and is described herein with specific reference to that application, but it will be appreciated that there are many other applications in which the invention could advantageously be employed.

There are many occasions in medical practice when fluids, such as blood, glucose or saline solution for example, are to be administered to the body and it is desirable that such administration takes place at a controlled metered rate. Medical infusion sets for administering fluid to the body commonly comprise a catheter, which is implanted in the patient, which is connected to a reservoir of the fluid by means of flexible tubing. In such infusion sets, it is known to provide some sort of adjustable clamp with which to pinch the flexible tubing and thereby achieve some measure of control over the fluid flow rate through the tubing. Other, more sophisticated techniques are also known, some of these being in the form of complicated valve mechanisms.

The present invention has the object of providing an improved apparatus for flow regulation without the disadvantages of conventional apparatus.

This object is achieved in that there is provided apparatus for flow regulation characterised by a housing including means for anchoring flexible tubing and means rotatable relative to the housing for applying a twist to the flexible tubing for controlling a flow of fluent material therethrough, the twisting means being spaced from the anchoring means, and including means for retaining the twisting means in any rotational position.

By way of example, some embodiments of flow regulating apparatus according to the present invention will now be described with reference to the drawings in which:

Figure 1 shows a cross-sectional view of one embodiment of flow regulating apparatus according to the invention,

Figure 2 shows a modified embodiment of the apparatus shown in Figure 1, and

Figure 3 shows a perspective view of a second embodiment of flow regulating apparatus.

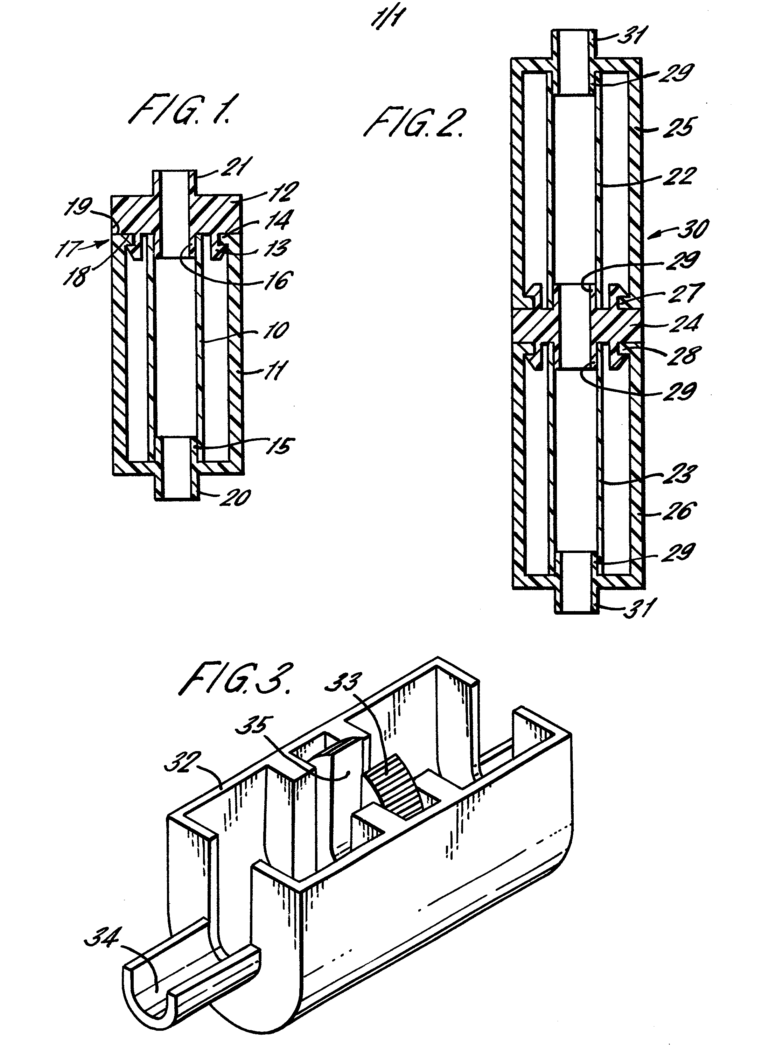

The apparatus shown in Figure 1 comprises a length of flexible tubing 10 extending between the ends of a cylinder 11. At one end of the cylinder 11, an annular disc 12 is rotatably mounted, and the disc is retained on the cylinder by means of a flanged spigot 13 which engages with a shoulder 14 on the inner surface of the cylinder. The disc, and possibly also the cylinder, is made of such material to have sufficient resilience to enable this snap-type assembly to be effected. It will be appreciated, however, that there are alternative ways of retaining the disc on the cylinder which could equally well be used here. The flexible tube 10 is mounted on a tubular protrusion 15 on the cylinder 11 at one end and on a similar tubular protrusion 16 on the disc 12 at the other, and in each case is secured by suitable means, for example, by application of a bonding agent. For regulating the flow of fluent material through the flexible tube using the apparatus shown in this embodiment, the disc is rotated with respect to the cylinder to create a twist in the flexible tube and thereby cause a constriction in the bore of the tube. ·

In order to control the rotation of the disc 12 with respect to the cylinder 11, a rachet-type arrangement 17 is provided between adjoining surfaces of the disc and the cylinder. A pattern of radially extending grooves can be formed on the end surface 18 of the cylinder 11 which register with a similar pattern of radially extending teeth formed on the surface 19 of the disc 12. Due to the resilient nature of the material of which the disc is made, it is possible to rotate the disc with respect to the cylinder. Once having rotated the disc to a desired setting, however, the ratchet-type arrangement ensures that the desired setting is maintained despite the torsional restoring force exerted by the twisted flexible tube. It will be understood that although a particular ratchet-type assembly has been described, there are a number of other ways in which control of the rotation of the disc could equally well be achieved.

Further tubular protrusions 20 and 21 are provided on the cylinder 11 and disc 12 respectively so that the flow regulating apparatus can be connected into a flow line, such as the tubing of a medical infusion set, for example. Again, the flow line can be secured to these protrusions using suitable means, as mentioned above. It will be noted that it is not essential here for both sections of the flow line, between which sections this apparatus is inserted, to be of flexible tubing.

The apparatus shown in Figure 2 comprises two lengths of flexible tubing 22 and 23, each extending between an annular disc 24 and the respective ends 25 and 26 of a cylinder 30. The ends 25 and 26 of the cylinder 30 are fixed with respect to each other, and the disc 24 is rotatably mounted in the intermediate portion between the ends of the cylinder. The disc 24 is located between intermediate shoulders 27 and 28 on the cylinder 30 and there is a similar ratchet-type arrangement between adjoining surfaces of the disc and the cylinder shoulders as has been described above. Each end of each length of flexible tubing 22 and 23 is received on a tubular protrusion 29 on the respective members and is secured thereon by suitable means as mentioned above.

For regulating the flow of fluent material through the flexible tubes in the apparatus shown in this embodiment, the disc is rotated with respect to the cylinder to create an equal and opposite twist in each of the lengths of flexible tubing causing a constriction in each of their bores.

Further protrusions 31 are provided at the ends of the cylinder 30 so that the apparatus can be connected into a flow line, such as the tubing of a medical infusion set, for example. Again, the flow line can be secured to these protrusions using suitable means, as mentioned above.

It will be noted that this flow regulating apparatus can be installed in a flow line which does not comprise flexible tubing. Furthermore, where this apparatus is installed in a flow line which does comprise flexible tubing, operation of the apparatus does not create any twisting of the flow line outwith the apparatus.

The apparatus shown in Figure 3 comprises a generally U-shaped elongate housing 32 in which is rotatably mounted a slotted annular disc 33. At either end of the housing 32 there is a

7.

semicylindrical seat 34 for a purpose to be described below. Between the disc 33 and the housing 32 there is a ratchet-type arrangement, of the same general sort as described above, with which to control the rotation of the disc with respect to the housing. The apparatus of this embodiment is designed so that it can be installed externally onto flexible tubing, such as may be found in medical infusion sets, for example, thus obviating the need for cutting into the tubing of an existing flow line. The flexible tube is laid in the housing 32 and secured to the semicylindrical seats 34 at each end of the housing by suitable means, as described earlier. At the same time, the flexible tube is laid in the slot 35 of the disc 33 to be secured in the bore of the disc in similar manner.

Again, by rotating the disc with respect to the housing, an equal and opposite twist is created in each portion of the flexible tube lying between the disc and a semicylindrical seat, causing a constriction in the bore of the tube. In this embodiment again, as in the embodiment shown in Figure 2, operation of the apparatus does not create any twisting of the flexible tube outwith the apparatus.

0105989

These embodiments of flow regulating apparatus allow the rate of fluid flow through the apparatus to be regulated between a full bore rate, in which the tubing is not twisted at all, and a zero flow rate in which the tubing is twisted so that its bore is fully occluded.

The embodiment of flow regulating apparatus shown in Figure 3 is particularly advantageous in medical applications in that the apparatus can be installed onto an existing flow line, and therefore contamination of the existing flow line is avoided, as indeed is the necessity for further sterilisation of the apparatus after installation.

Further advantages of these embodiments lie in the fact that the twisting of the tube can be arranged so that the twisted tube rests in tension and not in compression as is the case with brown clamp type arrangements. The "cold-flow" of the tubing may be minimised in this way.

9.

0105989

CLAIMS

1. Apparatus for flow regulation characterised by a housing (11) including means (15) for anchoring flexible tubing (10) and means (12) rotatable relative to the housing for applying a twist to the flexible tubing for controlling a flow of fluent material therethrough, the twisting means (12) being spaced from the anchoring means (15), and including means for retaining the twisting means in any rotational position.

2. Apparatus as claimed in claim 1 wherein the twisting means (12) is rotatable about the axis of the flexible tubing (10) so that twisting of the flexible tubing takes place about substantially its own axis.

3. Apparatus as claimed in claim 1 or claim 2 wherein the twisting means (12) is rotatably mounted on the housing (11) and the means for retaining the twisting means in any rotational position comprises a ratchet-type assembly operable between the twisting means and the housing.

4.    Apparatus as claimed in any preceding claim wherein the housing (11) is a cylindrical member and the twisting means (12) comprises an annular disc, the annular disc being mounted at one end of the cylindrical member, the flexible tubing (10) at one end thereof being anchored at the second end of the cylindrical member and at the other end thereof being secured to the annular disc.

5.    Apparatus as claimed in any one of claims 1 to 3 wherein the housing (11) is a cylindrical member and the twisting means (12) comprises an annular disc, the annular disc being mounted at an intermediate portion of the cylindrical member between the ends thereof, the flexible tubing (10) extending in individual lengths between the annular disc and a respective end of the cylindrical member, each individual length of flexible tubing at one end thereof being anchored to the respective end of the cylindrical member and at the other end thereof being secured to the annular disc.

6.    Apparatus as claimed in claim 4 or claim 5 wherein the anchored end of the flexible tubing (10) is anchored on the cylindrical member by being fastened onto an inwardly extending tubular spigot (15) on the respective end of the cylindrical member (11).

7. Apparatus as claimed in claim 4, claim 5 or claim 6 wherein the secured end of the flexible tubing (10) is secured to the annular disc (12) by being fastened onto a tubular extension (16) on the annular disc.

8. Apparatus as claimed in any one of claims 1 to 3 wherein the housing (32) is a generally U-shaped elongate member and the twisting means (33) comprises a slotted disc, the slotted disc being mounted at an intermediate portion of the elongate member between the ends thereof, the flexible tubing being laid into the elongate member and the slotted disc, and being anchored at the ends (34) of the elongate member, and being secured to the slotted disc.

9. Apparatus as claimed in claim 8 wherein the flexible tubing is anchored to the elongate member (32) and secured to the slotted disc (33) by means of a bonding agent.

FIG. 1.

FIG. 2.

FIG. 3.

0105989

1/1

**European Patent Office**

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-2 844 351 (SMITH) <br> * Column 2, lines 12-41; figures * | 1-3 | A 61 M 5/14 <br> F 16 K 7/08 |
| A | | 4 | |
| | --- | | |
| X | DE-A-2 216 480 (AQUA FEM) <br><br> * Page 14, line 13 - page 15, line 8; figures 11,12 * | 1,2,4 6,7 | |
| A | | 3 | |
| | --- | | |
| X | US-A-3 329 390 (HULSEY) <br> * Column 2, lines 33-72; figures 1,2 * | 1,2,4 | |
| A | | 6,7 | TECHNICAL FIELDS SEARCHED (Int. Cl. 3) <br><br> A 61 M <br> F 16 K |
| | --- | | |
| A | MACHINE DESIGN, vol. 23, no. 3, March 1951, page 112 <br> "Rubber sleeves" * Page 112 * | 5 | |
| | ----- | | |

The present search report has been drawn up for all claims

| Place of search <br> THE HAGUE | Date of completion of the search <br> 13-06-1983 | Examiner <br> VANRUNXT J.M.A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82